# NOUVEAU FASCICULE DE BREVET EUROPEEN

(11) **EP 0 905 138 B2**
(45) Date de publication et mention de la décision concernant l'opposition: **23.07.2008**
(45) Mention de la délivrance du brevet: 13.11.2002
(21) Numéro de dépôt: 98402334.1
(22) Date de dépôt: 23.09.1998
(51) Int. Cl.: C07H 15/04, A23L 1/236

(54) **Cristaux de maltitol de formes particulières, compositions cristallines les contenant et procédés pour leur préparation**
Speziell geformte Maltitolkristalle, diese enthaltende kristalline Zusammensetzungen und Verfahren zur ihrer Herstellung
Crystals of maltitol of a particular form, crystalline compositions containing them and processes for their preparation

(30) Priorité: 26.09.1997 FR 9712035
(43) Date de publication de la demande: 31.03.1999
(73) Titulaire: Roquette Frères, 62136 Lestrem (FR)
(72) Inventeur: Leleu, Jean Bernard, 62136 Lestrem (FR); Haon, Patrick, 67500 Haguenau (FR); Duflot, Pierrick, 62136 Lacouture (FR); Looten, Philippe, 59130 Lambersart (FR)
(74) Mandataire: Boulinguiez, Didier

(56) Documents cités:
- EP-A- 0 202 165
- EP-A- 0 735 042
- EP-A- 0 741 140
- US-A- 4 408 041
- US-A- 4 846 139
- US-A- 5 462 864
- US-A- 5 580 601
- US-A- 5 583 215
- US-A- 5 651 829
- S. OHNO, ET AL.: "X-Ray crystal structure of maltitol (4-O-alpha-D-glucopyranosyl-D-Glucitol)" CARBOHYDRATE RESEARCH, vol. 108, 1 janvier 1982, pages 163-171, XP000572365 AMSTERDAM, NL

## Description

La présente invention concerne l'utilisation de maltotriitol pour modifier ou contrôler la forme de cristaux de maltitol.

Pendant très longtemps, le maltitol n'a été présenté que sous forme de sirops de faible richesse.

Puis, le maltitol a été commercialisé sous forme de poudres amorphes et impures.

A la connaissance de la Demanderesse, ce n'est que vers 1980 que l'on a mis en évidence des cristaux de maltitol. Auparavant, ce polyol n'était pas connu comme formant facilement des cristaux.

La seule forme cristalline connue à ce jour pour le maltitol est la forme anhydre, laquelle a fait l'objet d'une protection large par brevet de la part de la société HAYASHIBARA (US 4.408.041).

Les techniques dites de "massé" d'une part et de cristallisation dans l'eau d'autre part, sont aujourd'hui quasiment les seuls procédés employés industriellement. Les produits ainsi obtenus, dont la cristallinité est très variable, ne conviennent pas tous particulièrement bien à certaines applications comme celles du chewing-gum ou du chocolat.

En outre, il est d'autres applications où ces produits ne sont pas totalement satisfaisants. C'est le cas par exemple lorsque l'on souhaite utiliser du maltitol pour remplacer le saccharose ou le lactose dans les formes sèches pharmaceutiques telles que les gélules, les médicaments du type poudres à dissoudre, les comprimés et les préparations nutritives pulvérulentes à diluer. C'est également le cas lorsque l'on souhaite réaliser le même genre de substitution dans les aliments sucrés tels que les boissons en poudre, les entremets, les préparations pour gâteaux ou les poudres chocolatées ou vanillées pour petit déjeuner.

On constate pour ces applications particulières, notamment pour les poudres pseudo-cristallines de maltitol obtenues par la technique de "massé" et à un degré moindre pour les poudres cristallines de maltitol obtenues par cristallisation dans l'eau, que celles-ci présentent un ou plusieurs défauts comme en particulier ceux de s'écouler difficilement, d'être sujettes à une prise en masse ou à un mottage, de ne se dissoudre que très lentement dans l'eau, d'être de mauvais excipients pour compression ou de ne pas satisfaire aux critères d'identification et de pureté imposés par différentes pharmacopées.

Désireuse d'améliorer l'état de la technique, la Demanderesse, a donc cherché à mettre au point des compositions de maltitol n'ayant pas les défauts d'écoulement, de mottage, de dissolution, ou de compression que présentent les poudres de maltitol connues. Certes, on aurait pu penser que le besoin identifié puisse être satisfait par d'autres polyols. Or, on constate qu'il n'en est rien car aucun d'entre eux ne possède des caractéristiques de solubilité, d'hygroscopicité, de saveur sucrée et de fusion aussi proches du saccharose que le maltitol.

Et c'est en travaillant sur la mise au point de ces compositions que la Demanderesse a pu isoler, de manière surprenante et inattendue, deux formes particulières de cristaux de maltitol, l'une bipyramidale et l'autre prismatique.

Il est du mérite de la Demanderesse d'avoir réussi, après avoir mené une recherche approfondie, à expliquer l'existence de ces deux formes de cristaux de maltitol. Elle a en effet mis en évidence que, contre toute attente, la forme des cristaux de maltitol était fonction de la teneur en maltotriitol d'un sirop de maltitol destiné à être cristallisé. La Demanderesse a constaté qu'en contrôlant la teneur en maltotriitol d'un sirop de maltitol, il était possible d'orienter la forme des cristaux de maltitol vers l'une ou l'autre des formes ou vers un mélange des deux formes, lorsque ce sirop de maltitol est soumis à une étape de cristallisation.

En conséquence, l'invention a trait l'utilisation de maltotriitol pour modifier ou contrôler la forme de cristaux de maltitol. Selon un premier aspect, les cristaux sont de forme bipyramidale comprenant deux tétraèdres réguliers juxtaposés par leur base de section carrée de 50 à 500 µm environ de côté, constituant ainsi des octaèdres réguliers d'environ 50 à à 500 µm de longueur d'arête.

Selon un deuxième aspect, les cristaux sont de forme prismatique se terminant par des faces planes constituant un tétraèdre, et qu'ils ont une longueur de 100 à 400 µm et une largeur d'environ 20 à 100 µm.

Les formes de cristallisation (bipyramidales ou prismatiques) ont nécessairement des conséquences importantes tant au niveau de la fabrication que des applications. Ainsi, une masse semi-cristallisée de maltitol, comprenant un certain pourcentage de cristaux prismatiques est plus visqueuse qu'une masse comprenant le même pourcentage de cristaux bipyramidaux, toute chose étant égale par ailleurs, et cela notamment lorsque les cristaux sont de taille importante.

C'est ainsi que pour préparer du maltitol atomisé, il est préférable de retenir des suspensions très pauvres en maltotriitol et comprenant de plus des cristaux bipyramidaux plutôt que prismatiques pour éviter un empâtement. Par ailleurs, l'utilisation de cristaux de maltitol bipyramidaux s'avère intéressante pour la production de chocolat (masse plus liée avant raffinage), de chewing-gums (possibilité de conserver une texture souple avec une quantité élevée de maltitol pulvérulent), de formes sèches pharmaceutiques (meilleur écoulement) etc ...

A contrario, une forme prismatique est plus compressible et permet un empâtement (prise en masse) à faible teneur en cristaux, recherché parfois (pâtes à mâcher, centres de chewing-gum à dragéifier).

D'autres caractéristiques et avantages de l'invention apparaîtront clairement à la lecture de la description qui suit, faite en référence aux dessins annexés, dans lesquels :
- les figures 1 et 2 représentent des photographies au microscope électronique à balayage de cristaux de forme bipyramidale conforme à l'invention ;
- la figure 3 représente une photographie au microscope électronique à balayage à moindre grossissement de cristaux identiques à ceux des figures 1 et 2 ;
- la figure 4 représente une photographie au microscope électronique à balayage d'un cristal de forme prismatique conforme à l'invention.

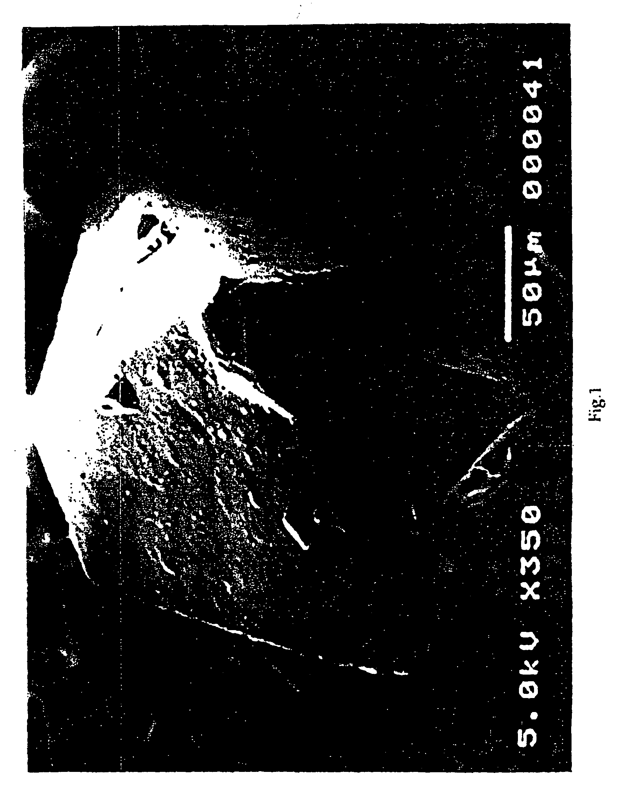

Les cristaux bipyramidaux et prismatiques sont illustrés aux figures 1 à 4.

Les observations ont été réalisées à l'aide d'un microscope électronique à balayage JEOL 5410, après avoir métallisé les cristaux à l'or grâce à un métalliseur JEOL JFC 1100 E (épaisseur de la couche d'or 100 Angstroms).

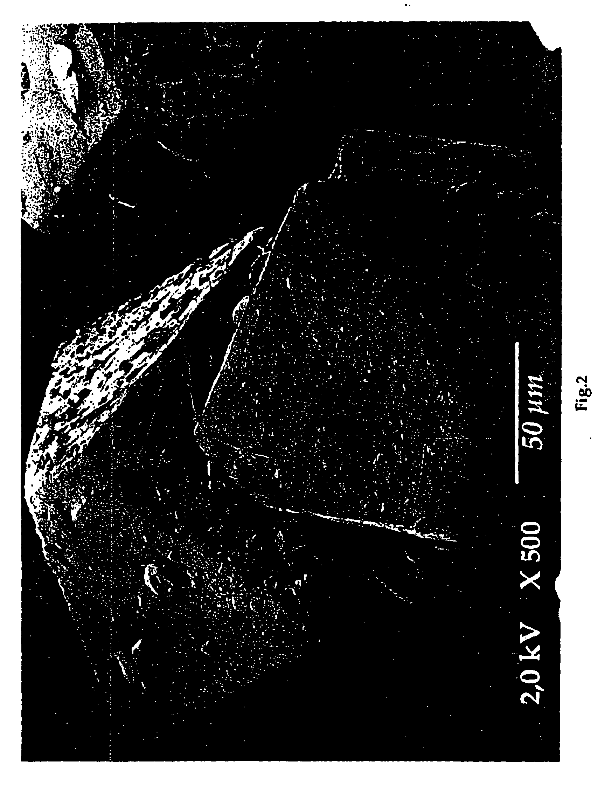

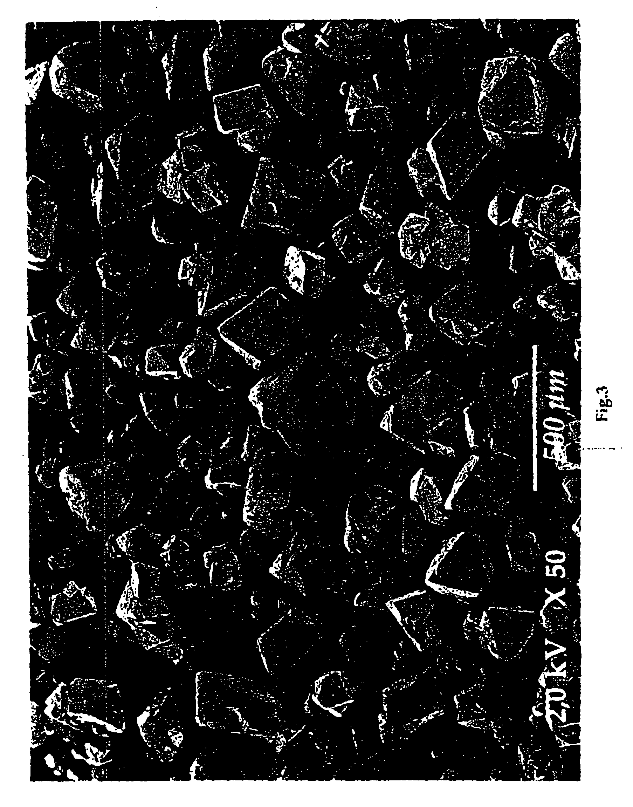

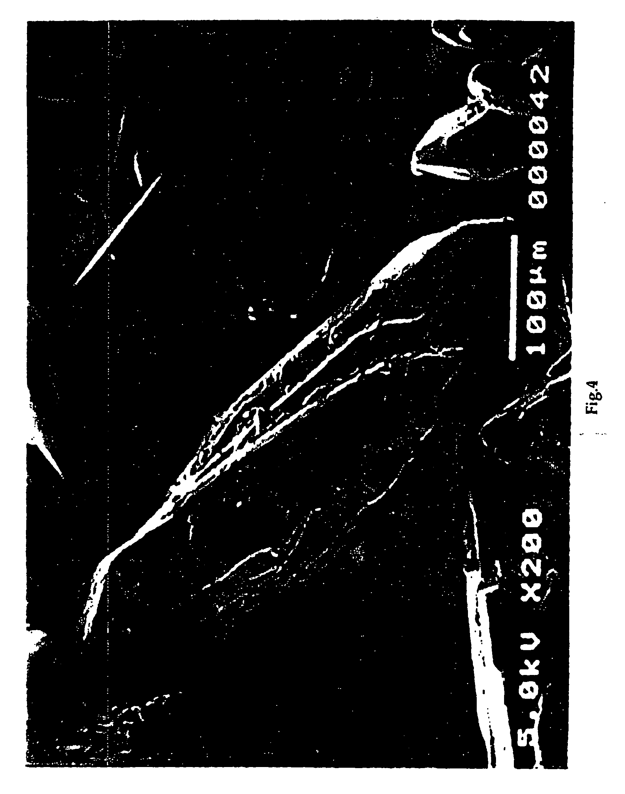

Les cristaux ont été observés sous une tension de 2 et de 5 kV. Les photographies sont captées sur le microscope avec un grossissement de 350 fois (figures 1), de 500 fois (figure 2), de 50 fois (figure 3) et de 200 fois (figure 4) puis agrandies lors de l'impression. Toutefois, une échelle reste inscrite sur la photographie et indique ainsi la taille réelle des cristaux.

Les cristaux des figures 1 et 2 sont de forme massive, bipyramidale. Plus précisément, ils ont la forme de deux trétraèdres réguliers, juxtaposés par leur base de section carrée, de 50 à 500 µm environ de côté, constituant ainsi des octaèdres réguliers d'environ 50 à 500 µm de longueur d'arête.

La figure 3 montre que les cristaux conformes à l'invention ne sont pas agglomérés ou organisés en petits paquets agglutinés mais sont au contraire bien dissociés et individualisés les uns par rapport aux autres.

Le cristal de la figure 4 apparaît comme un bâtonnet se terminant en pointe. Plus précisément, il a une forme prismatique dont la longueur est plus importante que la largeur (approximativement 5 fois plus importante que la largeur), se terminant par des faces planes constituant un tétraèdre. Ce bâtonnet a une longueur d'environ 100 à 400 µm sur une largeur d'environ 20 à 100 µm.

La composition cristalline de maltitol est constituée :
- soit de cristaux bipyramidaux conformes à l'invention ;
- soit de cristaux prismatiques conformes à l'invention ;
- soit à la fois de cristaux bipyramidaux et prismatiques.

La première caractéristique essentielle des compositions de maltitol tient au fait qu'elles sont cristallisées, ce qui leur confère une très haute stabilité vis-à-vis de l'humidité. Elles ont par conséquent une faible tendance à prendre en masse ou à motter. Aussi, leur usage est-il aisé et il n'est pas impératif de prendre des dispositions draconiennes pour prévenir ce risque.

Ces compositions cristallines de maltitol présentent toutes une richesse en maltitol supérieure ou égale à 87 %, de préférence supérieure ou égale à 92 %, et plus préférentiellement supérieure ou égale à 96 %, et avantageusement une teneur réduite en maltosyl-1,6 maltitol.

Ce qui les différencie essentiellement les unes des autres, c'est la teneur en maltotriitol.

Ainsi, lorsque la composition cristalline de maltitol est constituée de cristaux de maltitol de forme bipyramidale, elle présente une teneur en maltotriitol, en poids de matière sèche, inférieure à 1 %.

Lorsque la composition cristalline de maltitol est constituée de cristaux de maltitol de forme prismatique, elle présente une teneur en maltotriitol, en poids de matière sèche, supérieure ou égale à 4 %.

Et lorsque la composition cristalline de maltitol est constituée à la fois de cristaux de maltitol de forme bipyramidale et de forme prismatique, elle présente une teneur en maltotriitol, en poids de matière sèche, comprise entre 1 et 4 %.

La notion de richesse doit être entendue, dans le cas de la présente invention, comme correspondant au pourcentage de maltitol exprimé en poids sec/sec par rapport à l'ensemble des carbohydrates présents dans la composition cristalline de maltitol. Les carbohydrates peuvent être des polyols tels qu'en particulier le sorbitol, le maltotriitol et le maltotétraitol.

Les compositions cristallines de maltitol peuvent contenir, sans que leur présence altère de façon significative la cristallinité de ces compositions, certaines substances telles que par exemple des édulcorants intenses, des colorants, des pigments, des parfums, des arômes, des vitamines, des minéraux, des oligo-éléments, des principes actifs pharmaceutiques ou vétérinaires, des esters d'acides gras, des acides organiques ou minéraux et leurs sels, des matières protéiques comme les protéines, les acides aminés et les enzymes.

Les compositions cristallines de maltitol sont susceptibles d'être obtenues par cristallisation d'un sirop de maltitol présentant une richesse en maltitol supérieure ou égale à 87 %, de préférence supérieure ou égale à 92 % et plus préférentiellement supérieure ou égale à 96 % et une teneur en maltotriitol qui, selon la composition que l'on désire obtenir, est inférieure à 1 %, comprise entre 1 et 4 %, ou supérieure 4 % en poids de matière sèche.

L'une des caractéristiques essentielles de l'invention est donc de faire varier les teneurs en maltotriitol des sirops de maltitol cristalliser tout en maintenant avantageusement une teneur réduite en maltosyl-1,6 maltitol.

Pour orienter la forme des cristaux de maltitol, on contrôle la teneur en maltotriitol du sirop de maltitol à cristalliser. Ce contrôle de la teneur en maltotriitol du sirop de maltitol à cristalliser peut être effectuée en amont et/ou en aval de l'étape de cristallisation.

En amont de l'étape de cristallisation :
- au niveau de la fabrication du sirop de maltose en mettant en oeuvre des enzymes hydrolysant le maltotriose, et/ou
- en effectuant un tamisage moléculaire du sirop de maltose destiné à être hydrogéné puis cristallisé, et/ou
- en effectuant un tamisage moléculaire du sirop de maltitol destiné à être cristallisé, et/ou
- en effectuant une hydrolyse enzymatique du sirop de maltitol destiné à être cristallisé.

En aval de l'étape de cristallisation :
- en redissolvant la composition cristalline de maltitol dans l'eau et en effectuant un tamisage moléculaire sur le sirop ainsi obtenu et/ou une hydrolyse enzymatique, et/ou
- en redissolvant la composition cristalline de maltitol dans l'eau et en y ajoutant les quantités de maltotriitol nécessaires pour obtenir, après recristallisation, une nouvelle composition cristalline de maltitol présentant la teneur désirée en maltotriitol.

Toutes ces possibilités de contrôle de la teneur en maltotriitol peuvent être utilisées seules ou en combinaison les unes avec les autres.

De ce qui précède, il ressort donc que, conformément à l'invention, on peut orienter la forme des cristaux de maltitol, ce qui présente une très grande souplesse d'utilisation. Ceci permet en effet de passer indifféremment de cristaux de forme bipyramidale à des cristaux de forme prismatique et réciproquement.

Pour préparer le sirop de maltitol, on met en oeuvre le procédé décrit ci-dessous ou un procédé équivalent.

La première étape du procédé est en soi connue. Elle consiste à liquéfier un lait d'amidon dont l'origine botanique peut être quelconque : il peut provenir du blé, du maïs ou de la pomme de terre par exemple.

Ce lait d'amidon ou de fécule est additionné d'acide dans le cas d'une liquéfaction dite acide, ou d'une α-amylase dans le cas d'une liquéfaction enzymatique.

Dans le procédé de préparation du sirop de maltitol qui permet d'obtenir les compositions conformes à l'invention, on préfère effectuer une hydrolyse ménagée du lait d'amidon de façon à obtenir un lait d'amidon liquéfié à faible taux de transformation. Ainsi, les conditions de température, de pH, de taux d'enzyme et de calcium, connues de l'homme du métier, sont déterminées de manière telle qu'elles permettent d'obtenir un DE (Dextrose Equivalent) inférieur à 10, de préférence inférieur à 6, et plus particulièrement inférieur à 4.

De préférence, l'étape de liquéfaction est conduite en deux sous-étapes, la première consistant à chauffer, pendant quelques minutes et à une température comprise entre 105 et 108°C, le lait d'amidon en présence d'une α-amylase (type TERMAMYL^{R} 120L commercialisée par la société NOVO) et d'un activateur à base de calcium, la seconde consistant à chauffer le lait d'amidon ainsi traité à une température comprise entre 95 et 100°C pendant une à deux heures.

Une fois l'étape de liquéfaction terminée, dans les conditions de teneur en matières sèches, de pH, de taux d'enzyme et de calcium bien connues de l'homme du métier, on procède à l'inhibition de l'α-amylase. Cette inhibition de l'α-amylase peut se faire de préférence par voie thermique, en procédant en sortie de liquéfaction à un choc thermique de quelques secondes à une température supérieure ou égale à 130°C.

On effectue ensuite l'étape de saccharification. Lors de cette étape, on soumet tout d'abord le lait d'amidon liquéfié à l'action d'une α-amylase maltogénique, telle que celle commercialisée par la société NOVO, sous le nom Maltogénase^{R}. Lors de cette première étape de saccharification, l'α-amylase maltogénique peut être ajoutée en une seule fois ou en plusieurs fois.

A ce stade du procédé, il est déjà possible de contrôler la teneur en maltotriose (qui après hydrogénation conduit au maltotriitol) formé au cours de l'hydrolyse de l'amidon, en ajustant la quantité d'α-amylase maltogénique en fonction de la teneur en maltotriose et donc de la forme des cristaux de maltitol que l'on souhaite obtenir.

On poursuit ensuite, après avoir laissé agir l'α-amylase maltogénique, la saccharification du lait d'amidon liquéfié au moyen d'une β-amylase telle que celle commercialisée par la société GENENCOR sous la dénomination SPEZYME^{R} BBA 1500.

Lors de ces étapes, il convient d'associer aux enzymes ayant une activité maltogénique (α-amylase maltogénique et β-amylase) une enzyme hydrolysant spécifiquement les liaisons α-1,6 de l'amidon. Cet ajout d'une enzyme débranchante permet d'une part d'accélérer les réactions d'hydrolyse sans simultanément accélérer les réactions de reversion et, d'autre part, de réduire la quantité d'oligosaccharides hautement branchés résistant normalement à l'action des enzymes maltogéniques.

Cet ajout d'enzyme débranchante peut se faire au moment de l'ajout de l'α-amylase maltogénique ou au moment de l'ajout de la β-amylase.

Cette enzyme débranchante est choisie dans le groupe constitué par les pullulanases et les isoamylases.

La pullulanase est, par exemple, celle commercialisée par la société ABM sous la dénomination PULLUZYME^{R}.

L'isoamylase est, par exemple, celle commercialisée par la société HAYASHIBARA.

Avantageusement, le procédé est mis en oeuvre en présence d'isoamylase pour laquelle la société demanderesse a constaté qu'elle permettait non seulement d'obtenir un sirop de maltose présentant une teneur en maltose plus élevée qu'en utilisant une pullulanase, mais aussi d'obtenir un sirop de maltose présentant une teneur réduite en maltosyl-1,6 maltose et donc en maltosyl-1,6 maltitol après hydrogénation.

L'étape de saccharification peut être conduite également totalement ou partiellement en présence d'α-amylase fongique.

En fin de saccharification, il est possible d'ajouter un peu d'α-amylase, ce qui améliore généralement les étapes subséquentes de filtration. Les quantités et les conditions d'action des différentes enzymes mises en oeuvre dans les étapes de liquéfaction et de saccharification du lait d'amidon sont généralement celles qui sont recommandées pour l'hydrolyse de l'amidon et sont bien connues de l'homme du métier.

On effectue la saccharification jusqu'à ce que l'hydrolysat de maltose contienne au moins 87 %, de préférence au moins 92 %, et plus préférentiellement au moins 96 % en poids de maltose.

L'hydrolysat ainsi saccharifié est ensuite filtré sur filtre à précouche ou par microfiltration sur membranes, puis déminéralisé.

A ce stade du procédé, il est éventuellement possible d'effectuer sur cet hydrolysat saccharifié et purifié, une étape de cristallisation du maltose ou un tamisage moléculaire, cette étape de tamisage moléculaire permettant de contrôler la teneur en maltotriose du sirop de maltose, c'est-à-dire pour appauvrir plus ou moins, ou pas du tout, le sirop de maltose en maltotriose. Cette étape de tamisage moléculaire peut permettre ainsi de récupérer :
- soit une première fraction enrichie en maltose et oligosaccharides supérieurs et une seconde fraction enrichie en glucose ;
- soit une première fraction enrichie en oligosaccharides supérieurs et une seconde fraction enrichie en maltose et glucose ;
- soit, enfin, une première fraction enrichie en oligosaccharides supérieurs, une deuxième fraction enrichie en maltose et une troisième fraction enrichie en glucose.

Cette étape de tamisage moléculaire peut consister, par exemple, en une étape de séparation chromatographique ou en une étape de séparation sur membranes.

L'étape de fractionnement chromatographique est effectuée de manière connue en soi, de façon discontinue ou continue (lit mobile simulé), sur des adsorbants du type résines cationiques, ou sur des zéolithes fortement acides, chargées préférentiellement à l'aide d'ions alcalins ou alcalino-terreux tels que le calcium ou le magnésium mais plus préférentiellement à l'aide d'ions sodium.

En lieu et place de l'étape de séparation chromatographique, il est possible de mettre en oeuvre une étape de séparation par nanofiltration sur membranes. Des membranes de différents diamètres de pores sont fabriquées à partir de nombreux polymères et copolymères du type polysulfones, polyamides, polyacrylonitrates, polycarbonates, polyfuranes, etc.

Des exemples de l'utilisation de telles membranes sont décrits notamment dans les documents US-A-4.511.654, US-A-4.429.122 et WO-A-95/10627.

L'hydrolysat de maltose ainsi obtenu peut alors être facilement hydrogéné catalytiquement.

L'hydrogénation d'un tel hydrolysat s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de RANEY. On préfère cependant utiliser des catalyseurs au nickel de RANEY qui sont moins onéreux.

Dans la pratique, on utilise de 1 à 10 en poids de catalyseur par rapport à la matière sèche de l'hydrolysat soumis à l'hydrogénation. L'hydrogénation s'effectue de préférence sur un hydrolysat dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition de produits de cracking ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine le catalyseur par filtration et on déminéralise le sirop de maltitol ainsi obtenu sur des résines cationiques et anioniques. A ce stade, les sirops contiennent au moins 85 % de maltitol.

Selon un premier mode de réalisation du procédé, on met en oeuvre sur le sirop de maltitol obtenu à l'étape d'hydrogénation précédente, la succession des étapes consistant à :
- effectuer éventuellement un fractionnement chromatographique, connu en soi, de manière à obtenir une fraction riche en maltitol et une fraction plus ou moins riche en maltotriitol en fonction de la forme des cristaux désirée ;
- concentrer la fraction riche en maltitol ;
- cristalliser et séparer les cristaux de maltitol formés ;
- recycler les eaux-mères de cristallisation en amont de l'étape de fractionnement chromatographique.

Selon un deuxième mode de réalisation du procédé, on met en oeuvre sur le sirop de maltitol obtenu à l'étape d'hydrogénation précédente, la succession des étapes suivantes consistant à :
- concentrer le sirop de maltitol ;
- cristalliser et séparer les cristaux de maltitol formés.

Selon un troisième mode de réalisation du procédé, on met en oeuvre sur le sirop de maltitol obtenu à l'étape d'hydrogénation précédente, la succession des étapes suivantes consistant à :
- effectuer éventuellement une hydrolyse enzymatique du sirop de maltitol, au moyen par exemple d'une amyloglucosidase immobilisée ou non de manière à transformer le maltotriitol éventuellement présent en maltitol ;
- concentrer le sirop de maltitol ainsi obtenu ;
- cristalliser et séparer les cristaux de maltitol formés.

Selon un autre mode de réalisation du procédé, on met en oeuvre sur l'hydrolysat de maltose obtenu après saccharification la succession des étapes suivantes consistant à :
- effectuer éventuellement un fractionnement chromatographique, connu en soi, de manière à obtenir une fraction riche en maltose et plus ou moins riche en maltotriose ;
- hydrogéner la fraction riche en maltose ;
- cristalliser et séparer les cristaux de maltitol formés.

L'invention sera maintenant décrite à l'aide de l'exemple qui suit donné uniquement à titre illustratif et non limitatif.

### EXEMPLE

### 1. Conditions des essais

Le sirop à cristalliser est concentré à 80 % de matières sèches, placé dans un cristallisoir de laboratoire et stabilisé en température à 50°C, puis une amorce de MALTISORB^{R} (maltitol cristallisé commercialisé par la Demanderesse) à raison de 1 % / matière sèche, est ajoutée et le cristallisoir est refroidi sous agitation lente jusqu'à 20°C, à raison de 0,3°C par heure. Après turbinage et clairçage à l'éthanol, les cristaux sont séchés et observés au microscope électronique à balayage.

### 2. Résultats

Différentes bases sont mises en oeuvre ; leur composition et la forme des cristaux obtenus sont résumées dans le tableau suivant.

| **COMPOSITION** | **ASPECT DES CRISTAUX** |
|---|---|
| DP2H > 99 % | homogènes, de forme bipyramidale |
| | |
| DP2H : 93,5 % | hétérogènes, de formes bipyramidale et prismatique |
| DP3H : 3,8 % | |
| Sup. : 2,7 % | |
| DP1H : 5,2 % | |
| | |
| DP2H : 90,1 % | homogènes, de forme bipyramidale |
| DP3H : 0,9 % | |
| DP4H : 3,8 % | |
| | |
| DP2H : 96 % | homogènes, de forme prismatique |
| DP3H : 4 % | |
| DP1H = sorbitol | |
| DP2H = maltitol | |
| DP3H = maltotriitol | |
| DP4H = maltotétraitol | |
| Sup. = maltotétraitol et homologues supérieurs | |

## Revendications

1. Utilisation de maltotriitol pour modifier ou contrôler la forme des cristaux de maltitol.

## Claims

1. The use of maltotriitol to modify or control the form of maltitol crystals.

## Patentansprüche

1. Verwendung von Maltotriitol zur Modifizierung oder Steuerung der Form von Maltitolkristallen.
